# EUROPEAN PATENT SPECIFICATION

(11) **EP 2 227 252 B1**
(45) Date of publication and mention of the grant of the patent: **25.07.2012**
(21) Application number: 08855301.1
(22) Date of filing: 27.11.2008
(51) Int. Cl.: A61K 39/39, A61K 36/45, A61K 39/00, A61K 39/02, A61K 39/09, A61K 39/116, A61K 39/21

(54) **Adjuvant comprising vaccinium macrocarpon**
Adjuvans von Vaccinium macrocarpon
Adjuvant à base de vaccinium macrocarpon

(30) Priority: 27.11.2007 US 4418 P
(43) Date of publication of application: 15.09.2010
(73) Proprietor: Hochman, Nira, 96755 Jerusalem (IL); Hochman, Jacob, 96755 Jerusalem (IL); Weiss, Ervin, 46345 Herzeliya (IL); Ofek, Itzhak, 53464 Givataim (IL)
(72) Inventor: Hochman, Nira, 96755 Jerusalem (IL); Hochman, Jacob, 96755 Jerusalem (IL); Weiss, Ervin, 46345 Herzeliya (IL); Ofek, Itzhak, 53464 Givataim (IL)
(74) Representative: Duffy, Assumpta Dympna
(86) International application number: PCT/IL2008/001551
(87) International publication number: WO 2009/069130

(56) References cited:
- US-A1- 2005 196 472
- HOCHMAN NIRA ET AL: "Cranberry juice constituents impair lymphoma growth and augment the generation of antilymphoma antibodies in syngeneic mice." NUTRITION AND CANCER 2008, vol. 60, no. 4, 4 July 2008 (2008-07-04), pages 511-517, XP008106562 ISSN: 1532-7914
- STORNI T ET AL: "Immunity in response to particulate antigen-delivery systems" ADVANCED DRUG DELIVERY REVIEWS, ELSEVIER BV, AMSTERDAM, NL, vol. 57, no. 3, 10 January 2005 (2005-01-10), pages 333-355, XP025283918 ISSN: 0169-409X [retrieved on 2005-01-10]
- STEINBERG D ET AL: "Cranberry high molecular weight constituents promote Streptococcus sobrinus desorption from artificial biofilm" INTERNATIONAL JOURNAL OF ANTIMICROBIAL AGENTS, ELSEVIER SCIENCE, AMSTERDAM, NL, vol. 25, no. 3, 1 March 2005 (2005-03-01), pages 247-251, XP025326016 ISSN: 0924-8579 [retrieved on 2005-03-01]
- WEISS E I ET AL: "Cranberry juice constituents affect influenza virus adhesion and infectivity" ANTIVIRAL RESEARCH, ELSEVIER SCIENCE BV., AMSTERDAM, NL, vol. 66, no. 1, 1 April 2005 (2005-04-01), pages 9-12, XP004797873 ISSN: 0166-3542
- SHMUELY H ET AL: "Susceptibility of Helicobacter pylori isolates to the antiadhesion activity of a high-molecular-weight constituent of cranberry" DIAGNOSTIC MICROBIOLOGY AND INFECTIOUS DISEASES, ELSEVIER SCIENCE PUBLISHING CO., AMSTERDAM, NL, vol. 50, no. 4, 1 December 2004 (2004-12-01), pages 231-235, XP004669528 ISSN: 0732-8893
- LIPSON STEVEN M ET AL: "Cranberry cocktail juice, cranberry concentrates, and proanthocyanidins reduce reovirus infectivity titers in African green monkey kidney epithelial cell cultures" MOLECULAR NUTRITION & FOOD RESEARCH, WILEY - VCH VERLAG, WEINHEIM, DE, vol. 51, no. 6, 1 June 2007 (2007-06-01), pages 752-758, XP009108532 ISSN: 1613-4125
- DAVIES J ET AL: "A juicy problem" LANCET THE, LANCET LIMITED. LONDON, GB, vol. 358, no. 9299, 22 December 2001 (2001-12-22), page 2126, XP004805195 ISSN: 0140-6736

## Description

### FIELD OF THE INVENTION

The subject invention relates to the field of adjuvants.

### BACKGROUND

Infectious diseases have always been a scourge to human beings and their companion animals. Many infectious diseases are ubiquitous and often fatal. Vaccination is the most efficacious and valuable tool in the prevention of infectious diseases, provided that they are administered prophylactically in anticipation of pathogen exposure. Vaccines mainly capitalize the immune system's ability to respond rapidly to microorganisms after a second encounter and have been described as 'weapons of mass protection' (Cohen, J., Marshall, E., 2001, Science, 294(5542): 498-501*).*

The goal of vaccination is to stimulate a strong, protective and long-lasting immune response to the administered antigen. For the achievement of these objectives, potent adjuvant and novel vaccine strategies are required to make the vaccine sufficiently immunogenic to initiate a potent immune response. Currently, aluminium (Alum, a generic term for salts of aluminium) is an FDA-approved adjuvant. Comparative studies in humans and animals showed that aluminum is a weak adjuvant for antibody induction to recombinant protein vaccines and induces a Th2, rather than a Th1 response.

Cranberry (*Vaccinium macrocarpon* Ait. Ericaceae) fruit and juice have been reported to exert a multitude of health related benefits. Among these are the prevention of bacterial adhesion in urinary tract infections of *E*. *coli,* inhibition of *Helicobacter pylori* adhesion to human gastric mucus, inhibition of influenza virus adhesion and infectivity, antioxidant action, cholesterol reduction, effect on biofilm formation, and *in vitro* anti-cancer activity in a variety of cell lines.

NDM is a high molecular weight (> 12,000 daltons), non-dialysable material derived from cranberry juice as described in Ofek 1, Goldhar J and Sharon N. Anti-Escherichia coli adhesin activity of cranberry and blueberry juices. Adv Exp Med Biol 1996,408:179-183*,* US 6,303,125*,* US 5,840,322*, and* US 6,843,993.

Storni et al., Advanced Drug Delivery Reviews, Elsevier BV, Amsterdam, NL, Vol. 57, No. 3, 10 January 2005, pages 333 - 355, discloses certain adjuvants for vaccine use, but does not disclose or suggest an adjuvant of the type described and claimed herein.

### SUMMARY OF THE INVENTION

It has now been found that NDM based adjuvants have the unique ability to enhance immunity not only against proteins produced by pathogens (e.g. bacterial pathogens) but also against proteins produced by cancer cells.

NDM derived adjuvants can therefore enhance protection not only of vaccines against pathogens but also of vaccines against cancer cells.

The subject invention now provides a novel sub-fraction of NDM according to claim 12, named hereinafter NDM (12-30K), having a molecular weight of from about 12 to about 30 KDa;

The subject invention further provides an adjuvant according to claim 1 comprising NDM (12-30K). The subject invention also provides a use according to claim 3 of NDM (12-30K) for the manufacture of an adjuvant.

The subject invention also provides a vaccine comprising an adjuvant of the invention.

A vaccine of the invention may be used in a method of treating and preventing cancer comprising administering a vaccine of the invention.

A vaccine of the invention may be used in a method of preventing an infectious disease comprising administering a vaccine of the invention.

### FIGURES

Figure 1 - Growth inhibition curves of Rev-2-T-6 cells exposed to increasing concentrations ofNDM and NDM (12-30 K) derived from cranberry juice.
Figure 2 - Effect of cranberry juice fractions on in vitro invasion of Rev-2-T-6 lymphoma cells through extracellular matrix: A - NDM ; B - NDM (12-30K) *p<0.05, **p<0.01 statistical analysis one-way ANOVA with Dunnett's post-test comparing each to control of 0 treatment with NDM
Figure 3
   A) Western blot analysis using Rev-2-T-6 cell extract.
      lanes 1-4: Sera from tumor-bearing control mice inoculated with Rev-2- T-6 cells;
      lanes 5-8: Sera from tumor-free mice inoculated with Rev-2-T-6 cells followed by 4mg NDM (12-30K);
      lanes 9-10: Sera from tumor-free mice inoculated with Rev-2-T- 6 cells followed by 2mg NDM (12-30K);
      lanes 11-13: Sera from naïve Balb/C mice.
   B) Quantitation of Western Blot
      The lanes on the autoradiogram were digitized using a Fuji LS 3000 and quantitated using the Fuji Image Gauge V 3.46 program. Lanes 1, 5 & 10 are compared in the figure, but all lanes were averaged to compare the immunological reactivity. All sera were used at a 1:200 dilution.

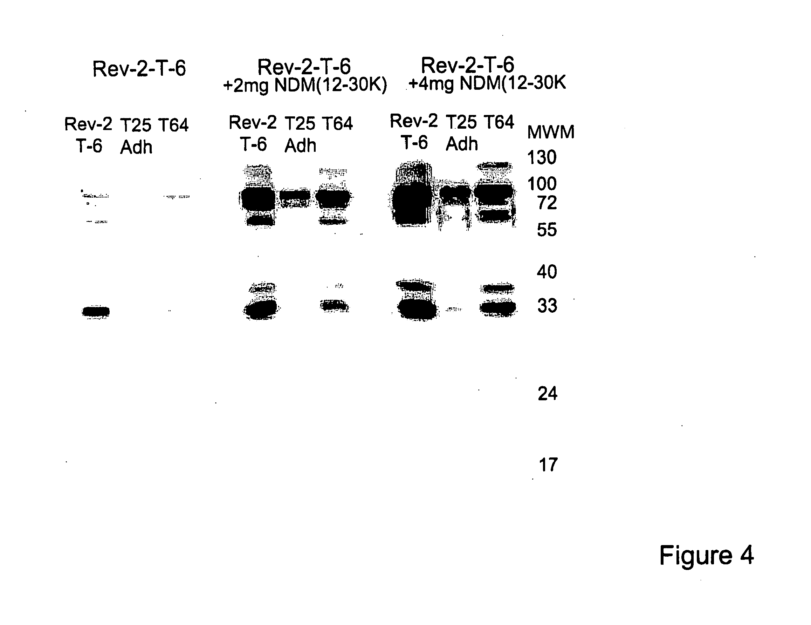

Figure 4 - Anti-Rev-2-T-6 antibodies from immunized mice recognize similar antigens in different S49 lymphoma cell variants. Rev-2-T-6, T-25-Adh and T-64 cells were lysed in SDS-PAGE sample buffer, run on 10% gels, and Western blotted versus sera from mice inoculated with Rev2-T-6 cells only, or with 2 or 4 mg of NDM(12-30K). Sera were used at 1:200 dilution.

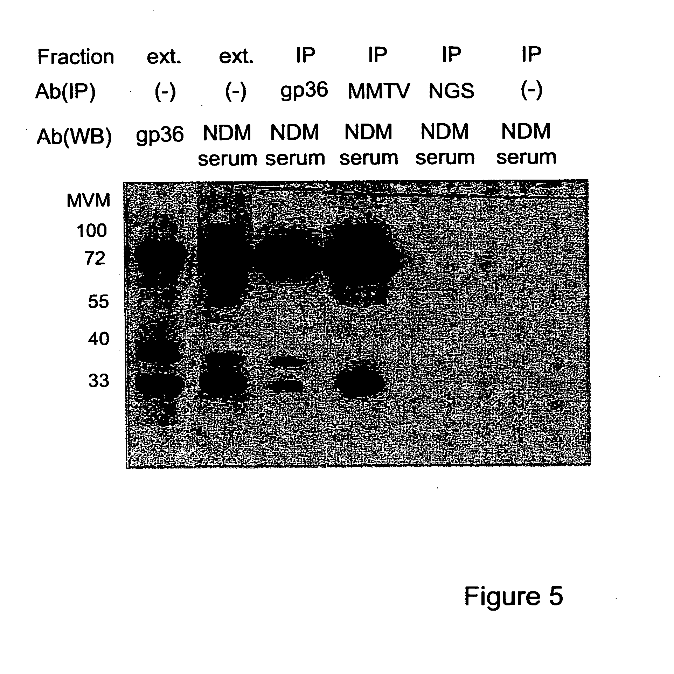

Figure 5 - Rev-2-T-6 cell extract using the indicated antibody was immunoprecipitated. Immunoprecipitated proteins were solubilized in SDS-PAGE sample buffer and run on 10% gels, blotted and probed with the appropriate antibodies. Ext. - Rev-2-T-6 whole cell extract; IP immunoprecipitate of Rev-2-T-6 cell extract; Ab(IP) Antibody used for immunoprecipitation; Ab(WB) Antibody used for western blot; gp36 - Goat-anti-MMTV-gp36; MMTV - Goat-anti-whole MMTV; NDM serum - serum from NDM (12-30K) immunized mice; NGS normal goat serum; MWM -molecular weight markers.

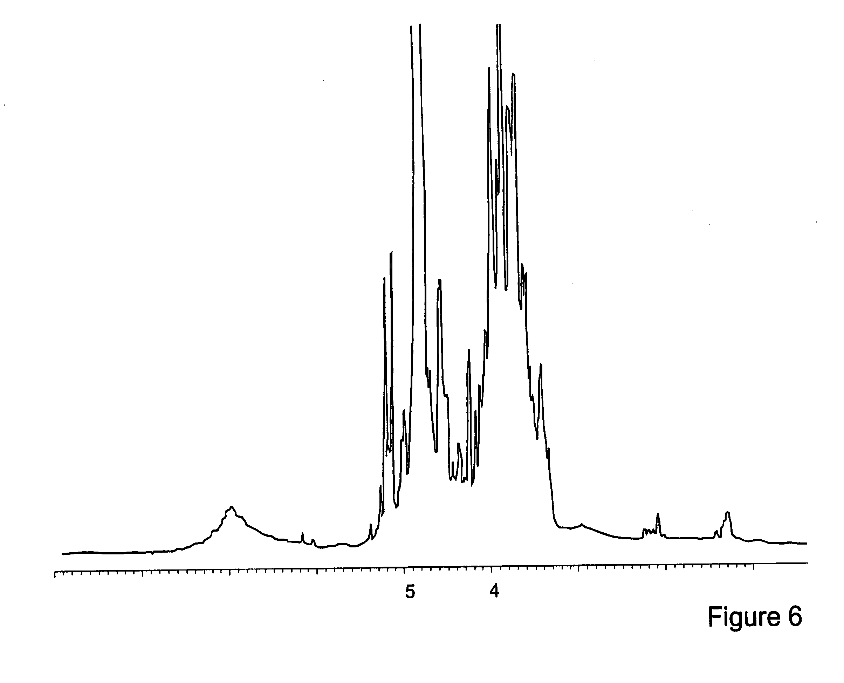

Figure 6- ¹H-NMR profile ofNDM (12-30K).
Figure 7 -
   Panel A - UV-VIS spectra of NDM and of NDM (12-30K);
   Panel B - comparison of copper-induced oxidation of NDM and NDM (12-30K).

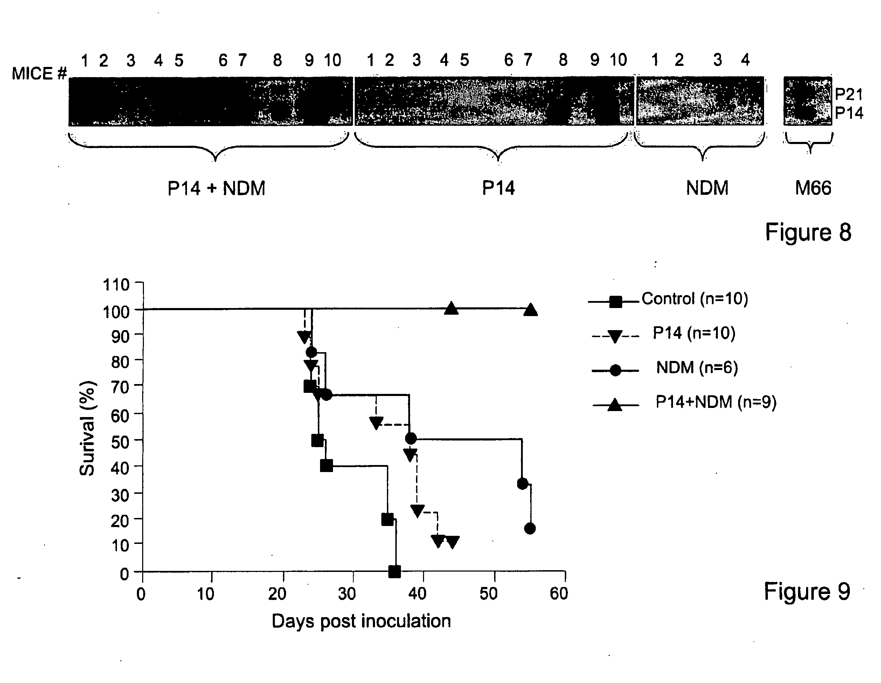

Figure 8 - Effect of NDM(12-30K) on antibody production in Balb/C mice.
   Mice were primed and boosted 3 times with p14+NDM(12-30K), with p14 alone and with NDM(12-30K) alone. Sera from individually bled mice at a dilution of 1:100 were subjected to western blotting using T-64 lymphoma cell extract. M-66 is a monoclonal antibody that recognizes both p14 and p21 (an extended version of p14) in extracts of T-64 cells and serves for positive control.
Figure 9 - NDM(12-30K) enhances the protection against lymphoma.
   Mice, previously primed and boosted with p14+NDM(12-30K), p14 alone and NDM(12-30K) alone were challenged with T-64 lymphoma cells. Naïve Balb/C mice inoculated with T-64 cells served as controls. Mice were followed for lymphoma development and survival.

### DETAILED DESCRIPTION

The subject invention provides a novel fraction of NDM named hereinafter NDM (12-30K) having a molecular weight of from about 12 to about 30 KDa. This fraction is obtainable by a process comprising (i) ultra filtration of concentrated Vaccinium macrocarpon extract through a membrane excluding 12-30 kD followed by (ii) dialysis at a molecular weight cut-off≥12,000.

The subject invention provides an adjuvant comprising NDM (12-30K) as defined above. The subject invention also provides a use of NDM (12-30K) as defined above for the manufacture of an adjuvant.

An adjuvant of the subject invention is isolated from the plant genus *Vaccinium ;* specifically, an adjuvant of the invention is isolated from cranberry.

An adjuvant of the subject invention may be obtained from the juice of cranberries as well as from concentrates of cranberry juice which may be prepared by methods known in the art and may be obtained from commercially available products.

An adjuvant is an agent that acts non-specifically to increase specific immune response or responses to an antigen. An adjuvant can act with a wide range of antigens, such as, but not limited to bacterial antigens, viral antigens, cancer antigens and so forth.

The subject invention further provides a vaccine comprising an adjuvant of the invention.

A vaccine as used herein is an antigenic preparation used to establish immunity to a disease. Vaccines can be prophylactic (e.g. to prevent or ameliorate the effects of a future infection by any natural or "wild" pathogen), or therapeutic (e.g. vaccines against cancer). Vaccines may be dead or inactivated organisms or purified products derived from them. There are four types of traditional vaccines:
- Vaccines containing killed microorganisms - these are previously virulent micro-organisms that have been killed with chemicals or heat.
- Vaccines containing live, attenuated microorganisms - these are live micro-organisms that have been cultivated under conditions that disable their virulent properties. They typically provoke more durable immunological responses and are the preferred type for healthy adults.
- Toxoids - these are inactivated toxic compounds from micro-organisms in cases where these (rather than the micro-organism itself) cause illness.
- Subunits - rather than introducing an inactivated or attenuated micro-organism to an immune system, a fragment of it can create an immune response.

A vaccine of the invention may be used in a method of treating and preventing cancer comprising administering a vaccine of the invention.

In one embodiment, an adjuvant of the invention is used for the treatment of cancer. The adjuvant may be administered alone, or in conjunction with, or as part of a vaccine to treat or prevent cancer.

The term "*cancer*" as used herein encompasses a neoplastic disease which is characterized by abnormal and uncontrolled cell division causing malignant growth or tumor. Cancer cells, unlike benign tumor cells, exhibit the properties of invasion and metastasis and are highly anaplastic.

In one embodiment of the present invention the cancer is a solid tumor or tumor metastasis.

In a further embodiment of the present invention, said cancer may be selected from, but is not limited to lung cancer (e.g. adenocarcinoma and including non-small cell lung cancer), pancreatic cancers (e.g. pancreatic carcinoma such as, for example exocrine pancreatic carcinoma), colon cancers (e.g. colorectal carcinomas, such as, for example, colon adenocarcinoma and colon adenoma), prostate cancer including the advanced disease, hematopoietic tumors of lymphoid lineage (e.g. acute lymphocytic leukemia, B-cell lymphoma, Burkitt's lymphoma), myeloid leukemias (for example, acute myelogenous leukemia (AML)), thyroid follicular cancer, myelodysplastic syndrome (MDS), tumors of mesenchymal origin (e.g. fibrosarcomas and rhabdomyosarcomas), melanomas, teratocarcinomas, neuroblastomas, gliomas, glioblastoma, benign tumor of the skin (e.g. keratoacanthomas), breast carcinoma (e.g. advanced breast cancer), kidney carcinoma, ovary carcinoma, bladder carcinoma and epidermal carcinoma. In one specific embodiment, the cancer is lymphoma. In another embodiment, the cancer is a cancer which harbors an MMTV virus such as a lymphoma and a carcinoma.

The term "*treating cancer*" as used herein relates e.g. to a decrease in tumor size; decrease in rate of tumor growth; stasis of tumor size; decrease in the number of metastasis; decrease in the number of additional metastasis; decrease in invasiveness of the cancer; decrease in the rate of progression of the tumor from one stage to the next, inhibition of tumor growth in a tissue of a mammal having a malignant cancer, control of establishment of metastases, inhibition of tumor metastases formation, regression of established tumors as well as decrease in the angiogenesis induced by the cancer. The term "*preventing cancer*" as used herein refers to prophylaxis such as prevention as cancer reoccurs after previous treatment (including surgical removal) and prevention of cancer in an individual prone (genetically, due to life style, chronic inflammation and so forth) to develop cancer.

A vaccine of the invention may further be used in a method of preventing an infectious disease comprising administering a vaccine of the invention.

In an embodiment, an adjuvant of the invention is used in conjunction with a vaccine aimed to prevent an infectious disease.

In a specific embodiment, the infectious disease is selected from the group consisting of a bacterial, viral, parasitic, fungal, helminthic and prion infection.

An infectious disease as used herein can be any infectious disease such as, but not limited to a bacterial infection, a viral infection, a parasitic infection, a fungal infection, a helmintic infection, a prion infection and so forth.

Non-limiting examples of such viral infectious diseases are AIDS, AIDS Related Complex, Chickenpox (Varicella),Common cold, Cytomegalovirus Infection, Colorado tick fever, Dengue fever, Ebola hemorrhagic fever, Hand, foot and mouth disease, Hepatitis, Herpes simplex, Herpes zoster, HPV, Influenza (Flu), Lassa fever, Measles, Marburg hemorrhagic fever, Infectious mononucleosis, Mumps, Poliomyelitis, Progressive multifocal leukencephalopathy, Rabies, Rubella, SARS, Smallpox (Variola), Viral encephalitis, Viral gastroenteritis, Viral meningitis, Viral pneumonia, West Nile disease, Yellow fever and so forth.

Non-limiting examples of such bacterial infectious diseases are Anthrax, Bacterial Meningitis, Botulism, Brucellosis, Campylobacteriosis, Cat Scratch Disease, Cholera, Diphtheria, Epidemic Typhus, Gonorrhea, Impetigo, Legionellosis, Leprosy (Hansen's Disease), Leptospirosis, Listeriosis, Lyme disease, Melioidosis, MRSA infection, Nocardiosis, Pertussis (Whooping Cough), Plague, Pneumococcal pneumonia, Psittacosis, Q fever, Rocky Mountain Spotted Fever (RMSF), Salmonellosis, Scarlet Fever, Shigellosis, Syphilis, Tetanus, Trachoma, Tuberculosis, Tularemia, Typhoid Fever, Typhus, Urinary Tract Infections and so forth.

Non-limiting examples of such parasitic infectious diseases are African trypanosomiasis, Amebiasis, Ascariasis, Babesiosis, Chagas Disease, Clonorchiasis, Cryptosporidiosis, Cysticercosis, Diphyllobothriasis, Dracunculiasis, Echinococcosis, Enterobiasis, Fascioliasis, Fasciolopsiasis, Filariasis, Free-living amebic infection, Giardiasis, Gnathostomiasis, Hymenolepiasis, Isosporiasis, Kala-azar, Leishmaniasis, Malaria, Metagonimiasis, Myiasis, Onchocerciasis, Pediculosis, Pinworm Infection, Scabies, Schistosomiasis, Taeniasis, Toxocariasis, Toxoplasmosis, Trichinellosis, Trichinosis, Trichuriasis, Trichomoniasis, Trypanosomiasis and so forth.

Non-limiting examples of such fungal infectious diseases are Aspergillosis, Blastomycosis, Candidiasis, Coccidioidomycosis, Cryptococcosis, Histoplasmosis, Tinea pedis and so forth

Non-limiting examples of such prion infectious diseases are transmissible spongiform encephalopathy, Bovine spongiform encephalopathy, Creutzfeldt-Jakob disease, Kuru-Fatal Familial Insomnia, Alpers Syndrome and so forth.

In one embodiment, the infectious disease is a bacterial infection of the respiratory tract. In another embodiment, the infectious disease is a bacterial urinary tract infection. In yet another embodiment, the infectious disease is an E. coli associated infection. In another embodiment, the *E.coli* associated infection results in diarrhea and meningitis.

Suitable routes of administration for an adjuvant or vaccine comprising an adjuvant of the subject invention are oral, rectal, nasal, topical (including transdermal, buccal and sublingual), vaginal or parenteral (including subcutaneous, intramuscular, intravenous and intradermal) administration or administration via an implant. In one embodiment, the adjuvant or vaccine comprising the adjuvant can be administered parenterally. In another embodiment, the adjuvant or vaccine comprising the adjuvant is administered by injection.

The exact dose and regimen of administration of an adjuvant or vaccine containing an adjuvant, will necessarily be dependent upon the therapeutic effect to be achieved

(treatment or prevention) and may vary with the route of administration, and the age and condition of the individual subject to whom the adjuvant is to be administered.

A dosage for humans is likely to contain from about 1 mg to about 1000 mg per dose. More specifically, and depending on the therapeutic effect to be achieved (e.g. treatment, prevention, administering a booster and so forth) and depending on whether an adjuvant or a vaccine comprising an adjuvant is administered, a dosage may range from 1-500mg, 1-300mg, 1-200mg, 2-80mg , 3-60mg, 4-40mg , 3-20mg, 2-10mg, 2-5mg and so forth. The desired dose may be presented as one dose or as multiple sub-doses administered at appropriate intervals, such as once a day, twice a day, every other day, once a week, once a month, once a year and so forth, depending on the therapeutic effect to be achieved (treatment, prevention, booster etc.)

An adjuvant of the invention may thus be administered in conjunction with, or comprised in, known vaccines such as, but not limited to, Cholera, Rabies, Tetanus, Typhoid fever, Bubonic plague, Diphteria,Periusis, Yellow fever, Typhus, Influenza, Polio,Japanese encephalitis, Anthrax, Adenovirus4&7, oral polio vaccine, Measles, Mumps, Rubella, Chicken pox, Pneumonia (Streptococcus pneumoniae), Meningitis(Neisseria meningitides), Human papilloma virus, Hepatitis A, Hepatitis B, Rotavirus, Lyme disease, Haemophilus influenza type b, Mycobacterium tuberculosis, Plasmid DNA and messenger RNA-based anti-cancer vaccination and so forth.

The present invention thus also relates to vaccines comprising an adjuvant of the subject invention in admixture with pharmaceutically acceptable auxiliaries, and optionally other therapeutic agents. The auxiliaries must be "acceptable" in the sense of being compatible with the other ingredients of the vaccine and not deleterious to the recipients thereof.

Adjuvants and/or vaccines include those suitable for oral, rectal, nasal, topical (including transdermal, buccal and sublingual), vaginal or parenteral (including subcutaneous, intramuscular, intravenous and intradermal) administration or administration via an . implant. The vaccines may be prepared by any method well known in the art of pharmacy.

Such methods include the step of bringing in association the active ingredient with any auxiliary agent. The auxiliary agent(s), also named accessory ingredient(s), include those conventional in the art, such as carriers, fillers, binders, diluents, disintegrants, lubricants, colorants, flavouring agents, anti-oxidants, and wetting agents.

Adjuvants and/or vaccines suitable for oral administration may be presented as discrete dosage units such as pills, tablets, dragées or capsules, or as a powder or granules, or as a solution or suspension. The active ingredient may also be presented as a bolus or paste. The adjuvant and/or vaccine can further be processed into a suppository or enema for rectal administration.

The invention further includes an adjuvant or a vaccine comprising an adjuvant, as hereinbefore described, in combination with packaging material, including instructions for the use of the adjuvant and/or vaccine for a use as hereinbefore described.

Adjuvants and/or vaccines suitable for parenteral administration include aqueous and non-aqueous sterile injection. The adjuvants and/or vaccines may be presented in unit-dose or multi-dose containers, for example sealed vials and ampoules, and may be stored in a freeze-dried (lyophilised) condition requiring only the addition of sterile liquid carrier, for example water, prior to use. For transdermal administration, e.g. gels, patches or sprays can be contemplated. Compositions or formulations suitable for pulmonary administration e.g. by nasal inhalation include fine dusts or mists which may be generated by means of metered dose pressurized aerosols, nebulisers or insufflators.

Adjuvants or vaccines of the invention can also be administered in the form of devices consisting of a core of active material, encased by a release rate-regulating membrane. Such implants are to be applied subcutaneously or locally, and will release the active ingredient at an approximately constant rate over relatively long periods of time, for instance from weeks to years. Methods for the preparation of implantable pharmaceutical devices as such are known in the art, for example as described in EP 303306.

Adjuvants and vaccines of the invention may be administered in conjunction with other compounds and compositions, including, but not limited to, estrogens, androgens, progestagens, antiprogestagens, chemotherapeutic agents such as cytotoxic and cytostatic agents, immunological modifiers such as interferons and interleukins, growth hormones or other cytokines, folic acid, vitamins, minerals and so forth, and/or in combination with surgery and/or radiation therapy.

The NDM(12-30K) fraction is obtainable by a process comprising ultra-filtering concentrated Vaccinium macrocarpon extract through a membrane excluding 12-30 kD followed by dialysing at a molecular weight cut-off≥12,0000

The invention is further described in the following examples, which are not in any way intended to limit the scope of the inventions as claimed.

### EXAMPLES

### General methods and materials

Cells: Rev-2-T-6 cells are lymphoma cells derived from S49 lymphoma which grow in vitro as clumps in suspension, and following intraperitoneal inoculation into mature, immune competent syngeneic mice, consistently develop as progressive solid tumors in 85%-95% of mice (Assaf N, et al, Virchows Arch 1997,-431(6):459-467*).* Upon IP inoculation into young mice, these cells also metastasize into the brain and eyes *(Assaf et al. supra;* Hochman et al., Cancer Res2001; 61 (13); 5242-5247*).*

Rev-2-T-6 and other lymphoma cell variants, T-64 and T-25-Adh *(*Braun E, Rorman E, Lueders KK, Bar-Sinai A and Hochman J. Differential expression of intracisternal A-particle transcripts in immunogenic versus tumorigenic S49 murine lymphoma cells. Virology 2000;277(1):136-146*),* were grown in Dulbecco's Modified Eagle Medium (DMEM), 10% horse serum, 50 units/ml penicillin and 0.05 mg/ml streptomycin (Biological Industries, Beit-Haemek, Israel) at 37°C in a humidified atmosphere containing 5% CO₂

**Cranberry extract:** Cranberry juice concentrate from the American cranberry, *Vaccinium macrocarpon,* was obtained from Ocean Spray, Inc.

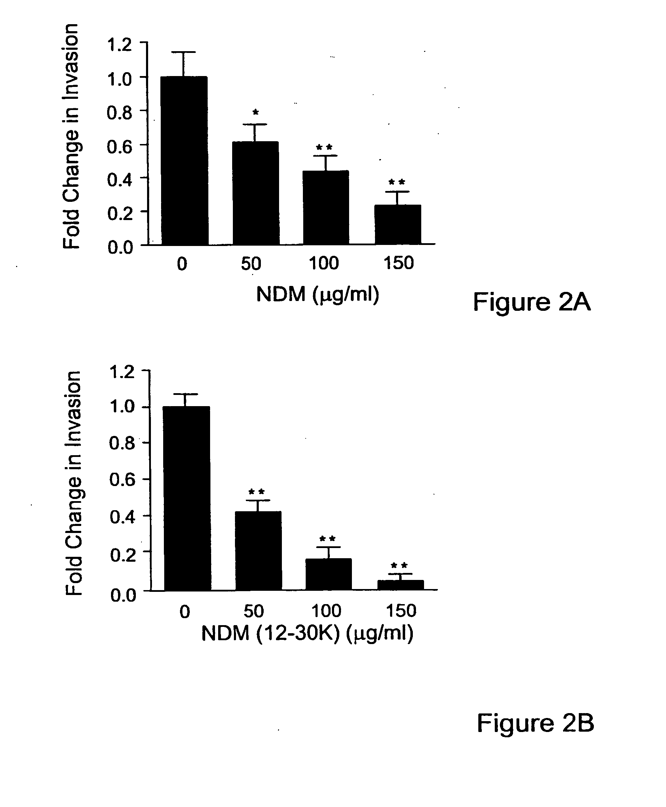

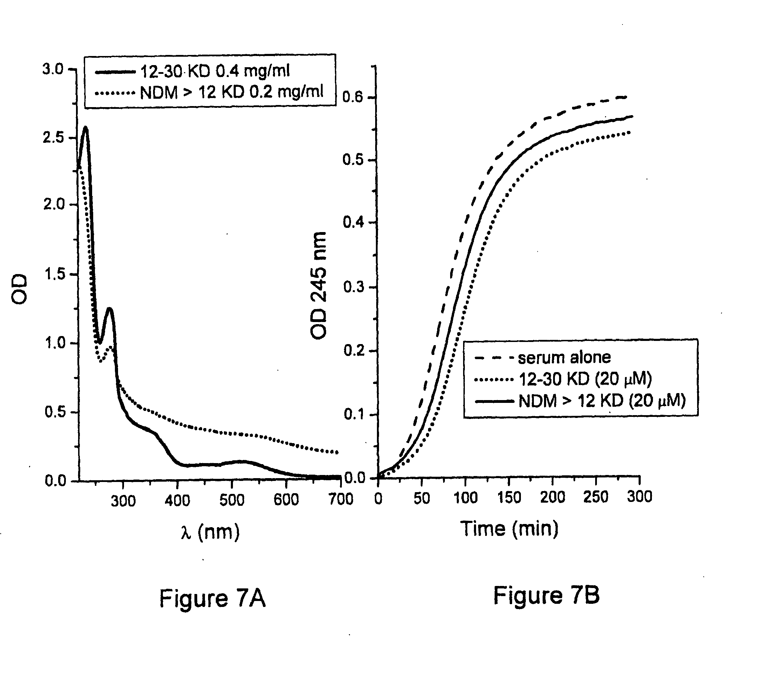

Two fractions were isolated from the concentrated juice:
(i) a fraction termed herein NDM and consisting of a non-dialyzable material (NDM) obtained as described in *Ofek et al. (1996), supra* by exhaustive dialysis of cranberry extract, at 4°C against distilled water in dialysis bags of 12,000 MW cut-off (Sigma - Aldrich, Corp. St. Louis, Missouri), followed by lyophilisation. NDM has an NMR line spectrum as set forth in Figures 2A and 2B of US 5,840, 322. NDM exhibits tannin-like properties, is highly soluble in water, devoid of proteins, carbohydrates and fatty acids and contains 56,6% carbon and 4,14% hydrogen *(*I. Ofek, Jet al., Adv. Exp. Med. Biol. 408:179-184*,* US 6,303,125*,* US 5,840,322*, and* US 6,843,993 all incorporated herein by reference in their entirety); and
(ii) a sub-fraction termed herein "NDM (12-30K)" and consisting of non-dialyzable material in the molecular weight range of 12,000-30,000 daltons obtained by two non-reversible separation steps: 1)ultra filtration (through YM 30; 76mm diameter membrane, Amicon, Millipore Corp., Bedford, MA) of concentrated cranberry extract followed by 2) dialysis followed by lyophilization as described above for the NDM fraction. When reversing the steps, i.e. first dialyzing and then ultrafiltrating, the NDM 12-30K fraction had a significantly lower adjuvant activity. NDM(12-30K) has the ¹H-NMR- spectrum as shown in Figure 6 and UV-VIS spectra as shown in Figure 7A.

***In vitro* proliferation assay:** Rev-2-T-6 cells (0.1x10⁶/ml) in tissue culture flasks, were incubated with NDM and NDM (12-30K), (50-250µg/ml) for 48hr. Cell proliferation was determined by hemacytometer counting as compared to control Rev-2-T-6 cells (w/o NDM). At least three replications for each sample were used. Growth inhibition curves and statistical analysis (standard deviation) were determined using GraphPad PRISM.

***In vitro* invasion assay:** In vitro invasion assays were performed as described *(*Albini A, Iwamoto Y, Kleinman HK Martin GR, Aaronson SA, Kozlowski JM, et al. A rapid in vitro assay for quantitating the invasive potential of tumor cells. Cancer Res 1987; 47(12):3239-3245*)* with the following modifications: the upper wells of BD Falcon HTS FluorBlok 24-multiwell inserts (8µm pore size, BD Biosciences, San Jose, CA) were coated with 100µl of Matrigel (0.5 mg/ml, Trevigen, Gaithersburg, MD) and dried overnight at 25°C. DMEM serum-free media conditioned for 24hr with NIH 3T3 cells and containing 0.1% BSA, was added to the lower chambers. Rev-2-T-6 cells were collected with phosphate-buffered saline (PBS) at 1x10⁶ cells/ml in serum-free DMEM containing 0.1% BSA and 200µl of cells were added to the upper chamber of the inserts. After 24 hr, the cells were stained with calcein AM (5µg/ml, Invitrogen) for 30 min at 37°C and read in a Wallac 1420 Victor2 multilabel plate reader (PerkinElmer, Shelton, CT). Each sample was measured in quadruplicate and each experiment was repeated three times. Data were analyzed using GraphPad PRISM and statistical analysis was performed using one-way anova with Dunnett's comparison for the post-test.

***In vivo* growth assay:** the following inoculation protocol for NDM (12-30K) was applied: Rev-2-T-6 cells (5x10⁶/mouse) were inoculated IP into Balb/C female mice 6-8 weeks old. NDM (12-30K) at 4mg per mouse (160mg/kg body weight), was injected IP one day after Rev-2-T-6 inoculation. Thereafter, the mice in this group were injected IP every other day, for two weeks, with 2mg NDM (12-30K) per mouse (80mg/kg body weight). The same pattern of inoculation was used for a second group of mice inoculated with Rev-2-T-6 cells, albeit with a first injection of 2mg NDM (12-30K) followed by 1mg (40mg/kg body weight) every other day for two weeks. Thus, the first group received a total of 18mg/mouse/experiment, and the second group received a total of 9mg/mouse/experiment. Control groups received either Rev-2-T-6 cells or NDM (12-30K) alone. Mice were followed daily for tumor development. Handling of mice was in accordance with NIH regulations.

**Western Blot analysis:** Cells were lysed by SDS-PAGE sample buffer, separated by 10% (or 15%) SDS-PAGE (Laemmli UK Cleavage of structural proteins during the assembly of the head of bacteriophage T4. Nature 1970; 227(5259):680-685*)* and transferred to nitrocellulose. After blocking for 1h with 5% non-fat dry milk in PBS containing Tween 20 (0.1%v/v), the nitrocellulose was incubated for 1h with either sera derived from individual mice, or polyclonal goat-anti MMTV. Following washing with PBS-Tween 20, the membrane was incubated for 1h with horseradish peroxidase-linked goat anti-mouse antibody or peroxidase-linked donkey anti-goat antibody (Jackson laboratory, Bar Harbor, ME). Nitrocellulose was washed again and ECL substrate (Amersham) was used for detection.

**Immunoprecipitation:** Cells were washed with PBS and extracted with 20mM phosphate lysis buffer (pH=7) containing 30 mM NaCl and a mixture of protease inhibitors (Sigma). Extracts were incubated on ice for 10 min, vortexed periodically, followed by 3 sequential 10 second sonications. Extracts were centrifuged at 10,000xg for 15 min at 4°C and precipitation buffer was added (150mM NaCl, 0.1% Triton X-100). Proteins were immunoprecipitated with goat anti-gp36, goat anti- MMTV or normal goat serum immobilized on protein G-Sepharose beads (GE Healthcare). Immune complexes were washed with washing buffer (0.1% Triton X- 100 in TBS, PH=7.5) and used for western blotting as described above.

### Example 1

### In vitro effects of cranberry juice constituents

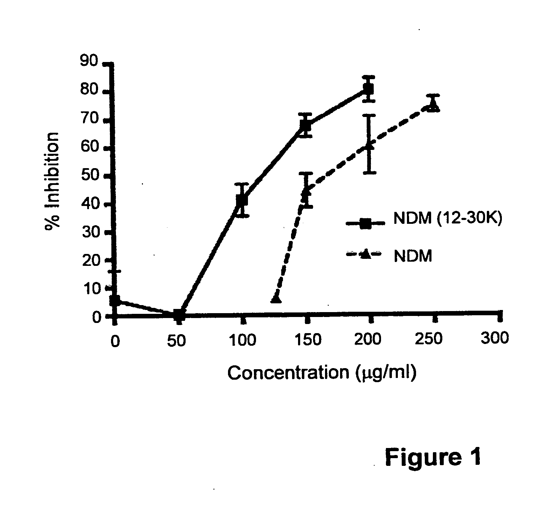

The ability of NDM and NDM (12-30K) to affect *in vitro* proliferation and invasion through extracellular matrix of Rev-2-T-6 cells was evaluated. Figure 1 depicts growth inhibition curves of Rev-2-T-6 cells exposed for 48hr to increasing concentrations of the two cranberry fractions. Both fractions are growth inhibitory. NDM (12-30K) is even more effective than NDM IC₅₀ 100 vs. 150µg/ml, respectively). NDM (12-30K) is also more effective in inhibiting the invasion of Rev-2-T-6 cells through extracellular matrix (Matrigel coated filters-see materials and methods) after 24hr (Fig. 2). This inhibitory effect is dose dependent and maximal at 150µg/ml NDM (12-30K). Furthermore, since NDM (12-30K) at 50µg/ml inhibits about 60% of cell invasion (Fig. 2B), while at the same concentration it has no growth inhibitory effect (Fig.1), the invasion inhibition effect is independent of the antiproliferative effect of NDM (12-30K). The same argumentation holds also for the NDM fraction. Therefore, these extracts may inhibit tumor development at concentrations below their cytotoxic threshold.

### Example 2

### In vivo inhibition of tumor growth

NDM (12-30K) was used for testing its efficacy in impairing Rev-2-T-6 cell growth in vivo in Balb/C female mice using the inoculation protocol described above. The results are shown in Table 1.

**Table 1: Effect of NDM(12-30K) cranberry juice constituent on Rev-2-T-6 lymphoma growth in Balb/C mice.**

| Treatment | Tumor development |
|---|---|
| Rev-2-T-6 cells | 4/5 (80%) |
| Rev-2-T-6 cells + NDM(12-30K) 2mg* | 0/5 (0%) |
| Rev-2-T-6 cells + NDM(12-30k) 4mg** | 0/4 (0%) |

Four out of five control mice inoculated with Rev-2-T-6 cells (80%) developed progressive tumors within 3 weeks post inoculation. These appeared as solid palpable abdominal masses. Mice with advanced tumors were bled and euthanized at 60 days post inoculation and tissues were collected for histopathological examination. Mice that were inoculated IP with Rev-2-T-6 cells, followed by IP inoculation of either 4 or 2mg NDM (12-30K) (see Table 1) were also bled at 60 days post inoculation. These mice demonstrated no signs of tumor development for 100 days when the experiment was terminated. Also, no tumors were found in these mice upon histopathological examination after 100 days. Thus, IP inoculation of NDM (12-30K) impairs the development of Rev-2-T-6 tumors. Naïve mice inoculated only with NDM (12-30K) showed no signs of stress. Also, NDM (12-30K) tested negative for endotoxin.

The findings that Rev-2-T-6 cells treated in vivo with NDM (12-30K) did not develop tumors even after 100 days post inoculation suggest that the tumors have been eradicated, either directly by NDM (12-30K) or indirectly by an activated immune system, following the growth retardation phase of Rev-2-T-6 cells.

### Example 3

### NDM (12-30K) elicits production of anti-tumor antibodies

Sera from the 3 different groups (Table 1) as well as from naïve Balb/C mice (at 1:200 dilution) were subjected to western blot analysis using Rev-2-T-6 cell extract. Exponentially growing Rev-2-T-6 cells were lysed in SDS- PAGE sample buffer and run on 10% gels, blotted and probed with sera from individual mice (see Table 1):

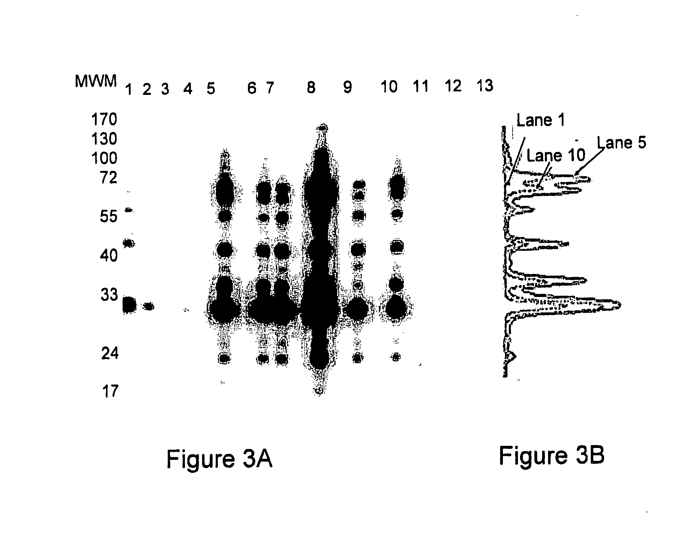

Figure 3 A demonstrates that mice inoculated with Rev-2-T-6 cells and NDM (12- 30K) generated anti-Rev-2-T-6 antibodies. All the sera from these mice demonstrate an identical pattern of antigen recognition. Mice inoculated only with Rev-2-T-6 cells show a very low titer of such antibodies, which varies between individual mice in this group. Naïve (non-inoculated) mice show no antibodies against Rev-2-T-6 cells.

These findings indicate that the NDM (12-30K) fraction derived from cranberry juice increases the immune response of mice towards Rev-2-T-6 cells, thereby impairing tumor growth in syngeneic hosts.

Figure 4 shows that lymphoma cell lines T-64 (highly tumorigenic, ascitic tumors, derived from the S49 T-cell lymphoma) and T-25-Adh (immunogenic, protection against a challenge with T-64 cells), from which Rev-2-T-6 cells were derived (*Braun et al., supra*), were also recognized by the above mentioned immune sera.

In an attempt to quantify the immune reactivity to the two doses of NDM(12-30K), all the lanes in the autoradiogram presented in Figure 3A were digitized (Fig. 3B) and the total area under the peaks quantitated. The ratio of the average reactivity of the sera from the animals which were treated with the 4mg NDM(12-30K) protocol to the average reactivity from animals which were treated with the 2mg protocol was 1.8. Similar results were obtained in all three lymphoma cell variants tested when the autoradiogram presented in Figure 4 was subjected to the same analysis (not shown). These findings are consistent with the antibody level in response to NDM(12-30K) being dose dependent.

### Example 4

### Antigens recognized by immunized mice are MMTV related

The antibodies raised against Rev-2-T-6 cells reacted not only with T-64 and T-25-Adh lymphoma cell variants, but also with Mm5mt and 4T1 murine mammary carcinoma cells. They did not react however with NIH-3T3 cells (not shown). A common denominator between the lymphoma and mammary carcinoma cell lines is their harboring of Mouse Mammary Tumor Virus - MMTV. NIH-3T3 cells are devoid of this virus. We therefore investigated whether the sera of immunized mice contained anti-MMTV antibodies. Immunoprecipitation of T-64 cell extract with anti-MMTV antibodies, followed by western blotting with sera from mice inoculated with lymphoma cells and NDM (12-30K), revealed that the major antigens recognized by these sera are indeed MMTV related (Fig.5).

### Example 5

Whole *S*. *pyogenes* bacteria (M5 group A streptococci , Manfredo strain), urinary isolate of *E.coli* (346) and M protein, a surface protein extracted and purified from *S*. *pyogenes* were employed.

The bacteria were cultivated as described in Ofek I et al., J Bacteriol. 154:139-145, 1983 *and* Perry , A., et al., Infect. Immun. 39:1334-1345, 1983*.* Briefly, bacteria were grown in brain heart infusion broth for 24 hours. The microorganisms were then span down and washed three times with sterile saline and adjusted to a density of 1.00 OD suspension.

M5 protein of *S*. *pyogenes* was extracted and purified as described in Ofek L, et al., J. Bacteriol. 149:426-433, 1982*.*

### Immunization

Antigens consisted of a suspension of 1.0OD of Group A Streptococci (GAS) and *E. coli* (EC) and 1 mg/ml M5 protein of *S*. *pyogenes.* Each antigen was injected to a group of 5 RIHS-Recombinant Inbred Heterogenous Strains (Kindly supplied by Dr. Fuad Iraqi, TelAviv University) The antigens were mixed with equal volume of either saline or saline supplemented with 5 mg/ml NDM. Thoroughly mixed and each mice received 0.2ml intraperitoneal (i.p.) of bacteria or intramuscular (i.m.) of 0.1ml of protein. On day 6 the mice were sacrificed, bled and the serum separated and kept at 4°C.

### Determination of antibody titer

Titer of bacterial antibodies was determined by slide agglutination test as follow: 20ul of 2.00 OD bacterial suspension mixed with serial two fold dilutions of 20ul mice serum on slides. The last dilution giving visible agglutination is recorded.

Titer of M5 protein antibodies were determined by ELISA as described in Hu M, Walls M, Stroop S, Reddish M, Beall B, Dale J. Immunogenicity of 26-valent group A streptococcal vaccine. Infect Immun 2002.2177-70:2171;

Briefly, serial two-fold dilution of mice serum starting at 1:10 were added to wells containing pre-coated with M protein in a microtiter plates. Last dilution giving 0.2 OD reading was recorded.

Table 2 shows that cranberry NDM injected with the indicated antigens enhanced the production of antibodies against *S*. *pyogenes* and *E.coli* as well as against the soluble bacterial M5 protein by several order of magnitudes as compared to antibody production in animals injected with the antigens alone.

**Table 2 - Adjuvant effect of NDM in mice immunized with i.p. bacteria and i.m. protein**

| Immunizing | Reciprocal Log₂ antibody titer of serum from mice number | | | | | | |
|---|---|---|---|---|---|---|---|
| Antigen | | #1 | #2 | #3 | #4 | #5 | Geometric Mean±SD |
| Non immunized | | ≤1 | ≤1 | | | | |
| | | | | | | | |
| GAS | | 2 | 3 | 2 | 4 | 2 | 2.6±0.9 |
| GAS+NDM | | 10 | 8 | 9 | 7 | 8 | 8.4±1.1 |
| | | | | | | | |
| EC | | 1 | 2 | 1 | 2 | 3 | 1.8±0.8 |
| EC+NDM | | 6 | 5 | 6 | 8 | 5 | 6.0±1.2 |
| | | | | | | | |
| M5 protein | | 7 | 7 | 8 | 10 | (IgM) | 8±1.4 |
| M5 protein +NDM | | 12 | 11 | 10 | 12 | (IgM) | 11.2±1.0 |

### Example 6

The specific activity of NDM(12-30K) on inhibition of inter-bacterial adhesion (coaggregation) of oral bacteria was compared to that of NDM. As shown in Table 1, the specific activity of the 12-30K is higher compared to that ofNDM.

**Table 1**

| | Coaggregation partners F. nucleatum - S. sanguis | Yield¹ |
|---|---|---|
| NDM | 0.35² | 1.0 |
| NDM(12-30K) | 0.18 | 0.25-0.35 |

The inhibitory effect of the cranberry fractions was assayed by visual scoring on a scale from 0 to 4 (according to Weiss EI, Shaniztki B, Dotan M, Ganeshkumar N, Kolenbrander PE, Metzger Z, Attachment of Fusobacterium nucleatum PK1594 to mammalian cells and its coaggregation with periodontopathogenic bacteria are mediated by the same galactose-binding adhesion, Oral Microbiol Immunol. 2000 Dec;15(6):371-7.
¹ Relative yield compared to NDM; (NDM is scored 1.0)
² Lowest concentration causing complete inhibition of coaggreagation (mg/ml).

### Example 7

UV-VIS spectra was measured using Kontron instruments (Via G. Fantoli 16/15 Milano Italy) (Uvikon 933A) double-beam UV/VIS spectrophotometer equipped with a 12-position automated sample changer.

Figure 7, Panel A shows UV-VIS spectra of NDM and of NDM (12-30K) demonstrating the common features of these two preparations in the UV-region (the most important being the peak at about 280 nm).

Figure 7, Panel B represents the comparison of the antioxidative potency of NDM and NDM (12-30K) as measured by copper-induced peroxidation.

The copper-induced peroxidation was monitored at 37°C by continuous recording of absorbance at 245 nm using a Kontron (Uvikon 933) double-beam spectrophotometer (Kontron Instruments, Milan, Italy) equipped with an automated 12-position sample changer. Measurements were carried out in quartz cuvettes (optical pathway 1 cm).

Absorbance at 245 nm (OD 245 nm) is mainly attributed to conjugated dienic hydroperoxides (conjugated dienes) and 7-ketocholesterol, the major products of lipid peroxidation. To trigger the free radical chain reaction, cupric chloride (CuCl₂, final concentration 100 µM) was added to a solution containing 720 µM sodium citrate in phosphate-buffered saline solution (146 mM NaCl, 3.3 mM NaH₂PO₄, 3.3 mM Na₂HPO₄, pH adjusted to 7.4) and 30 µL serum, in a final reaction volume of 1.5 mL.

Figure 7, Panel B thus shows the right shift of the kinetic curve of serum peroxidation in the presence of NDM (12-30K) which reflects that NDM(12-30K) has a higher antioxidative effect than NDM. The antioxidative effect depends on the operations implied for fraction isolation of fraction.

### Example 8

Three groups of Balb/C female mice were injected intra-muscularly at days: 1, 30, 75, and 110 as follows:
Group I: 75µg of antigen (N =10 mice);
Group II: 1 mg of adjuvant (N=6 mice);
Group III: 75µg antigen + 1mg adjuvant (N =10 mice).

The antigen used was the leader peptide of the envelope precursor protein of the virus MMTV (Mouse Mammary Tumor Virus), a 98 amino acid protein, shown in Western blotting analysis as a 14KDa protein and therefore named MMTV-p14, or p14 for short *(*Hoch-Marchaim H. Weiss A.M Bar-Sinai A. Fromer M Aderman K. and Hochman J.: The leader peptide of MMTV Env precursor localizes to the nucleoli in MMTV-derived T cell lymphomas and interacts with nucleolar protein B23. Virology 313: 22-32, 2003).

The adjuvant used was the NDM (12-30K) fraction.

No tissue lesions, nor apparent toxicity, nor impairment of mice mobility were observed throughout the experiment.

At day 126 (16 days after the last boost injection) mice were bled and individual sera (all at a dilution of 1:100) were subjected to Western blot analysis, using an extract of T-64 lymphoma cells that express both p14 and an extended version of p14 (named p21) expressed in these cells. For positive control we used a monoclonal antibody (M-66) that recognizes both p14 and p21 in T-64 cells.

Figure 8 shows that 8/10 mice (80%) injected with p14+NDM (12-30K)(Group III) demonstrated anti-p14 and p21 antibodies in their sera, while only 2/10 mice (20%) injected with p14 alone (Group I) revealed such antibodies. NDM(12-30K) alone (Group II) had no effect on anti-p14 or p21 production.

Thus, it is evident that NDM(12-30K) enhances the ability of p14 to generate antibodies specific to the p14 antigen and thus acts as adjuvant.

### Example 9

One week after bleeding (133 days after the first injection), mice from the groups of Example 8, as well as a control group of naïve mice, were inoculated intraperitoneally with 2.5X10⁶ T-64 lymphoma cells and followed thereafter (Figure 9). While none (0/10) of the control mice survived longer than 36 days, all mice (9/9) from the Group III previously injected with p14+NDM(12-30K) survived over 50 days post inoculation without any signs of tumor development. One out of 10 mice injected with p14 alone (Group 1), and 1 out of 6 mice injected with NDM(12-30K) alone (Group II) survived over 50 days. From these findings it is evident that NDM(12-30K) enhances the vaccination capacity of p14 to protect mice against a deadly challenge with a malignant lymphoma that expresses the p14 antigen.

## Claims

1. An adjuvant comprising a non dialyzable material (NDM) having a molecular weight of from 12 to 30 kDa and isolated from *Vaccinium macrocarpon,* said NDM being obtainable by a process comprising ultrafiltering a concentrated *Vaccinium macrocarpon* extract through a membrane excluding molecular weights of between 12 and 30 kDa; and dialysis of the filtered material at a molecular weight cut-off ≥12,000.

2. A vaccine comprising an adjuvant according to claim 1.

3. Use of a non dialyzable material (NDM) isolated from *vaccinium macrocarpon* and having a molecular weight of from 12 to 30 kDa for the manufacture of an adjuvant, said NDM being obtainable by a process comprising ultrafiltering a concentrated *Vaccinium macrocarpon* extract through a membrane excluding molecular weights of between 12 and 30 kDa; and dialysis of the filtered material at a molecular weight cut-off≥12,000.

4. The use according to claim 3 wherein the adjuvant is used for the treatment of cancer.

5. The use according to claim 4 wherein the cancer is lymphoma.

6. The use according to claim 3 wherein the adjuvant is used in conjunction with a vaccine aimed to prevent an infectious disease.

7. The use according to claim 6 wherein the infectious disease is selected from the group consisting of a bacterial, viral, parasitic, fungal, helminthic and a prion infection.

8. The use according to claim 7 wherein the infectious disease is a bacterial infection of the respiratory tract.

9. The use according to claim 7 wherein the infectious disease is a bacterial urinary tract infection.

10. The use according to claim 7 wherein the infectious disease is an E. coli infection.

11. The use according to claim 10 wherein the E. coli infection results in diarrhea and meningitis.

12. A fraction of a non dialyzable material (NDM) isolated from *Vaccinium macrocarpon* having a molecular weight of from 12 to 30 kDa, said fraction being obtainable by a process comprising ultrafiltering a concentrated *Vaccinium macrocarpon* extract through a membrane excluding molecular weights of between 12 and 30 kDa; and dialysis of the filtered material at a molecular weight cut-off ≥12,000.

## Patentansprüche

1. Adjuvans, das ein nichtdialysierbares Material (NDM) umfasst, das ein Molekulargewicht von 12 bis 30 kDa aufweist und aus *Vaccinium macrocarpon* isoliert wurde, wobei das genannte NDM durch ein Verfahren erhalten werden kann, das Folgendes umfasst: Ultrafiltration eines konzentrierten Extrakts aus *Vaccinium macrocarpon* durch eine Membran, die Molekulargewichte zwischen 12 und 30 kDa ausschließt; und Dialyse des gefilterten Materials bei einer Molekulargewichtsausschlussgrenze von ≥ 12 000.

2. Impfstoff, der ein Adjuvans nach Anspruch 1 umfasst.

3. Verwendung eines nichtdialysierbaren Materials (NDM), das aus *Vaccinium macrocarpon* isoliert wurde und ein Molekulargewicht von 12 bis 30 kDa aufweist, für die Herstellung eines Adjuvans, wobei das genannte NDM durch ein Verfahren erhalten werden kann, das Folgendes umfasst: Ultrafiltration eines konzentrierten Extrakts aus *Vaccinium macrocarpon* durch eine Membran, die Molekulargewichte zwischen 12 und 30 kDa ausschließt; und Dialyse des gefilterten Materials bei einer Molekulargewichtsausschlussgrenze von ≥ 12 000.

4. Verwendung nach Anspruch 3, worin das Adjuvans für die Behandlung von Krebs verwendet wird.

5. Verwendung nach Anspruch 4, worin der Krebs ein Lymphom ist.

6. Verwendung nach Anspruch 3, worin das Adjuvans zusammen mit einem Impfstoff verwendet wird, mit dem Ziel einer Infektionskrankheit vorzubeugen.

7. Verwendung nach Anspruch 6, worin die Infektionskrankheit aus der Gruppe ausgewählt ist, die aus einer bakteriellen, viralen, parasitären, Pilz-, Wurm- und einer Prionen-Infektion besteht.

8. Verwendung nach Anspruch 7, worin die Infektionskrankheit eine bakterielle Infektion des Respirationstrakts ist.

9. Verwendung nach Anspruch 7, worin die Infektionskrankheit eine bakterielle Hamwegsinfektion ist.

10. Verwendung nach Anspruch 7, worin die Infektionskrankheit eine Infektion mit E. coli ist.

11. Verwendung nach Anspruch 10, worin die Infektion mit E. coli zu Durchfall und Meningitis führt.

12. Fraktion eines nichtdialysierbaren Materials (NDM), das aus *Vaccinium macrocarpon* mit einem Molekulargewicht von 12 bis 30 kDa isoliert wurde, wobei die genannte Fraktion durch ein Verfahren erhalten werden kann, das Folgendes umfasst: Ultrafiltration eines konzentrierten Extrakts aus *Vaccinium macrocarpon* durch eine Membran, die Molekulargewichte zwischen 12 und 30 kDa ausschließt; und Dialyse des gefilterten Materials bei einer Molekulargewichtsausschlussgrenze von ≥ 12 000.

## Revendications

1. Adjuvant comprenant une matière non dialysable (NDM) ayant un poids moléculaire de 12 à 30 kDa et isolée à partir de *Vaccinium macrocarpon,* ladite NDM pouvant être obtenue par un procédé comprenant l'ultrafiltration d'un extrait de *Vaccinium macrocarpon* concentré à travers une membrane excluant des poids moléculaires compris entre 12 et 30 kDa ; et la dialyse de la matière filtrée à un seuil de rétention des molécules ≥ 12 000.

2. Vaccin comprenant un adjuvant selon la revendication 1.

3. Utilisation d'une matière non dialysable (NDM) isolée à partir de *Vaccinium macrocarpon* et ayant un poids moléculaire de 12 à 30 kDa pour la fabrication d'un adjuvant, ladite NDM pouvant être obtenue par un procédé comprenant l'ultrafiltration d'un extrait de *Vaccinium macrocarpon* concentré à travers une membrane excluant des poids moléculaires compris entre 12 et 30 kDa ; et la dialyse de la matière filtrée à un seuil de rétention des molécules ≥ 12 000.

4. Utilisation selon la revendication 3, dans laquelle l'adjuvant est utilisé pour le traitement du cancer.

5. Utilisation selon la revendication 4, dans laquelle le cancer est un lymphome.

6. Utilisation selon la revendication 3, dans laquelle l'adjuvant est utilisé conjointement avec un vaccin visant à empêcher une maladie infectieuse.

7. Utilisation selon la revendication 6, dans laquelle la maladie infectieuse est choisie dans le groupe constitué d'une infection bactérienne, virale, parasitaire, fongique, helminthique et à prion.

8. Utilisation selon la revendication 7, dans laquelle la maladie infectieuse est une infection bactérienne des voies respiratoires.

9. Utilisation selon la revendication 7, dans laquelle la maladie infectieuse est une infection bactérienne des voies urinaires.

10. Utilisation selon la revendication 7, dans laquelle la maladie infectieuse est une infection par E. coli.

11. Utilisation selon la revendication 10, dans laquelle l'infection par E. coli entraîne diarrhée et méningite.

12. Fraction d'une matière non dialysable (NDM) isolée à partir de *Vaccinium macrocarpon* ayant un poids moléculaire de 12 à 30 kDa, ladite fraction pouvant être obtenue par un procédé comprenant l'ultrafiltration d'un extrait de *Vaccinium macrocarpon* concentré à travers une membrane excluant des poids moléculaires compris entre 12 et 30 kDa ; et la dialyse de la matière filtrée à un seuil de rétention des molécules ≥ 12 000.
